# EUROPEAN PATENT APPLICATION

(11) **EP 4 449 975 A1**
(43) Date of publication of application: **23.10.2024**
(21) Application number: 21967966.9
(22) Date of filing: 15.12.2021
(51) Int. Cl.: A61B 3/08, A61B 3/113

(54) **GAZE DIRECTION TRACKING SYSTEM**

(71) Applicant: Neocortex Digital, S.L., 28001 Madrid (ES)
(72) Inventor: MARCOS GONZALEZ, Diego, 28001 MADRID (ES)
(74) Representative: Evens, Paul Jonathan
(86) International application number: PCT/ES2021/070891
(87) International publication number: WO 2023/111365

(57) **Abstract**

Disclosed is a system for tracking a user's gaze direction (3.5), which comprises: a plurality of light sources (2.1) that emit infrared light onto at least one eye (1) of the user; a camera (2.2) configured to capture infrared images of the eye (1); and a controller comprising at least one processor, wherein the controller is configured to detect a plurality of flashes in an image of the eye (1), captured by the camera (2.2), each flash being a reflection of one of the light sources (2.1) on the visible surface of the eye (1), and to match each detected flash to the light source (2.1) that generated the flash on the visible surface of the eye (1). According to the invention, the system is characterised in that: the plurality of light sources (2.1) is arranged in the form of a matrix covering, at least partly, the visible surface of the eye (1); and the controller is additionally configured to analyse the image of the eye (1) and to determine to which region of the eye (1) each pixel of the image belongs, generate a 3D shape of the eye (1) from the plurality of flashes relative to an absolute geometric reference determined by the position of the camera (2.2) and the plurality of light sources (2.1), and define an ocular centre of rotation (3.1) of the eye (1) on the basis of the 3D shape of the eye (1), with which the gaze direction (3.5) is determined.

## Description

### Technical field

The present invention relates to an ocular analysis system, more specifically it relates to a gaze direction tracking system.

### State of the art

Eye tracking is the process of evaluating either the point where the gaze is fixed (the place that is gazed at), or the rotational movement of the eye in relation to the head.

Eye-tracking systems, or eye-trackers, are known and widely used in commercial applications, such as: video games, hands-free interface for interaction with computers, analysis of the user's visual interest in complex environments, etc.

The gaze direction is the line that joins the active PRL (preferred retinal locus) with the centre of rotation of the eye. In a healthy person, said PRL is located in the fovea of the eye, while in people with various ocular pathologies (for example, with retinal pathologies such as AMD, scotoma, etc.) the PRL can be found outside the fovea.

It should be noted that a user can use more than one PRL, each in its position integral with the eye, but there can only be one active PRL at any given moment. This implies that the gaze direction, that is, the line that joins the active PRL with the ocular centre of rotation, changes when the user changes PRL. However, the measurement accuracy required to detect them exceeds what is available in the state of the art.

The most popular variant of these eye-tracking systems uses video images from which the eye position is estimated. Other methods use search coils or are based on electrooculography.

Gaze tracking systems that use video images typically comprise two or more infrared LEDs, to induce reflections on the visible surface of the user's eye (i.e., the cornea and/or sclera of the eye), and an infrared imaging camera to capture an image of the user's eye, including flares produced by such reflections.

Normally, when the camera is facing the user's eye, the LEDs, usually between 2 and 8, are located symmetrically on a flat crown around the camera.

The calculation of the gaze direction is carried out by a controller, with at least one processor, which collects the image captured by the infrared camera. Generally, this calculation is carried out image by image, the images being obtained under a constant infrared illumination system, and consists of determining the centre of rotation of the eye (through which it passes in the line of the gaze direction), as well as the contour of the pupil and the relative position of the flashes with respect to the contour of the pupil.

It should be noted that the image being analysed is a flat image (2D) that lacks information to detect depth, which makes these systems not very robust to variations in user position and its effects, specifically, variations in the distance from the eye and/or the distance from the pupil with the camera.

Given the limitations in the available information, these systems carry out their calculations taking some hypotheses that are far from reality, such as: i) that the user's pupil is always an ellipse; ii) that the pupil moves jointly with the eye; and iii) that the user's gaze direction passes, at all times, through the geometric centre of the pupil ellipse.

This is a crude approximation, which may cause an error of 0.5° to 1.5° (i.e., 30 to 90 arc minutes), when the physiological process to be measured, for example, drifting movements and microsaccades of the eye, occurs between 1 and 30 arc minutes.

All of this has the potential to generate a large error in the measurement of the gaze direction, either due to the calculation of the centre of rotation of the eye and/or because, in general, the gaze direction does not pass through the centre of the pupil, meaning that the known systems are insufficiently accurate.

It should be noted that, when the flashes of the infrared LEDs occur near the contour of the pupil, the flash does not allow quality detection of the contour of the pupil or determination of the geometric coordinates of the pupil with respect to that reference point, so that the quality of the information for the purposes of calculating the gaze direction deteriorates.

Likewise, when the user has a drooping eyelid or blinks, the top of the image and eventually some flares are obstructed, so the calculation of the geometric coordinates of the pupil is less accurate or cannot be performed.

Additionally, when the user changes their relative position with respect to the camera (because they breathe, cough, scratch, get distracted, etc.), the position reference between the camera and the eye is temporarily lost (in the worst case, the pupil of the eye may be outside the camera's field of view). In these cases, image quality is lost, further deteriorating the accuracy of the gaze direction tracking system.

Therefore, when the user changes their relative position with respect to the camera, recalibration is required to return to the initial measurement accuracy. Normally in these calibration processes, the user is asked to stare at a one-off stimulus of known coordinates with respect to the reference system. The user must maintain their gaze for a sufficient time on the stimulus, for example for 10 seconds, in order to determine the gaze direction by averaging a sufficient number of measurements.

It should be noted that, due to the natural visual dynamics, the user's eye produces, in that time, between 20 and 40 fixations along with other ocular movements, at least drifting and microsaccades, and eventually saccades, around the area in which the stimulus is shown introducing a significant error in the calibration.

The main reason for which this calibration follows this process is due to the normal magnitude of the measurement error, which requires statistical treatment, increasing the data acquisition time, to obtain satisfactory results.

Therefore, this calibration process is disruptive for the user, since they have to abandon the task they are doing to perform the calibration.

Therefore, a gaze direction tracking system is required that takes into account the true 3D shape of the eye, as well as reliably detects the contour of the pupil at all times, thereby obtaining more accurate results.

Likewise, a calibration process is required that is not disruptive for the user, allowing the user to continue with their activity.

### Object of the invention

Due to all of the foregoing, the object of the present invention is a user's gaze direction tracking system, which comprises:
- a plurality of light sources that emit infrared light onto at least one eye of the user;
- a camera configured to capture infrared images of the eye;
- a controller comprising at least one processor, wherein the controller is configured to:
   o detect a plurality of flashes in an image of the eye captured by the camera, each flash being a reflection of one of the light sources on the visible surface of the eye; and
   ∘ to match each detected flash to the light source that generated the flash on the visible surface of the eye;
characterised in that:
- the plurality of light sources is arranged in the form of a matrix covering at least partly the visible surface of the eye; and:
- the controller is additionally configured to:
   ∘ analyse the image of the eye and to determine to which region of the eye each pixel of the image belongs;
   ∘ generate a 3D shape of the eye from the plurality of flashes relative to an absolute geometric reference determined by the position of the camera and the plurality of light sources; and
   ∘ define an ocular centre of rotation of the eye on the basis of the 3D shape of the eye with which the gaze direction is determined.

That is, the plurality of light sources emit light towards an eye of the user, such that each light source generates flashes on the visible surface of the eye (either on the cornea or on the sclera of the eye). The camera then captures an image of the eye, said image being analysed by the controller, determining to which region of the eye (for example, pupil, iris, sclera, eyelid, eyelashes or corners) each pixel of the image belongs.

The plurality of light sources, preferably between 8 and 20 light sources, are arranged in the form of a matrix at different distances from each other vertically and horizontally, at least partly (or completely) covering the visible surface of the eye.

This matrix distribution of the plurality of light sources produces a distribution of flashes the correlative distances of which change depending on the depth of the region of the eye in which they occur, which will allow the 3D shape to be generated.

Since the distances between the plurality of light sources are known, each flash can be easily correlated with its corresponding light source. Producing a wide distribution of flashes across the surface of the eye reduces problems caused by blinking or drooping eyelids, as flashes are still visible in the image of the eye even under these conditions.

Likewise, by determining an absolute geometric reference, it is possible to triangulate the real position of each flash, allowing the controller to generate a 3D shape of the user's eye.

Since the eye behaves as a rigid body, the ocular centre of rotation is maintained in a constant position relative to the eye, when the controller compares two images of the eye corresponding to two gaze directions (for example, those obtained after a rotation of the eye), it can easily establish the ocular centre of rotation more accurately, which is critical for calculating gaze direction.

As the eye is maintained in a fixed position relative to the cranial structure, the movements detected in the position of the ocular centre of rotation with respect to the absolute geometric reference also describe the movements of the user's head in relation to this absolute geometric reference.

Preferably, the controller is additionally configured to:
- detect characteristic eye movements in a series of eye images captured by the camera during a gaze fixation,
- estimate a point outside the eye of the gaze direction line on the basis of the characteristic movements of the eye in said fixation;
- define an active eye PRL of the eye in the 3D shape of the eye as the point of intersection with the rear portion of the 3D shape of the eye of the gaze direction line defined by the ocular centre of rotation and the point outside the eye.

Fixation is the visual process by means of which the user positions the image of a stimulus on their active PRL and by means of which their visual system extracts information about the content in spatial frequencies (and other information, such as colour, etc.) of what the user sees. To obtain this information, the eye must move with a characteristic movement called drift, interrupted by other movements called microsaccades that reposition the image of the stimulus on the fovea.

To determine the gaze direction, the present system takes advantage of the characteristic movements of the eye during a fixation. These movements are involuntary and occur exclusively when there is a stimulus on which the user can fix their gaze. If, for example, this stimulus is unique in the user's virtual visual space and the user's eye shows fixation movements, it can be concluded that the user is fixating on that stimulus.

Since the PRL determined in this way is integral with the eye, it will serve for the automatic determination of the gaze direction as the direction of the axis that passes through the ocular centre of rotation and the active PRL.

Therefore, the determination of the gaze direction in the present system is defined as the line or axis that joins the active PRL (whether or not it is in the fovea) with the centre of rotation of the eye. This line is completely independent of the pupil and has no relation to the contour of the pupil nor does it have to pass through its centre.

By not determining the gaze direction artificially (such as in the state of the art where the gaze direction is defined as the line generated by the connection of the ocular centre of rotation with the centre of the pupil), with the present system much more accurate results are obtained than those obtained with eye tracking systems of the state of the art.

Preferably, the plurality of light sources comprises at least two groups of light sources configured to emit light alternately, i.e., when the light sources of one group are turned on, the rest of the light sources would be turned off.

In this way, it is possible to improve the image of the contour of the pupil of the eye, since if in an image captured by the camera, a flash from the active group of light sources makes it difficult to distinguish the contour of the pupil, turning on a second group, and turning off the first, improves the visibility of the pupil contour in the image.

This alternation between groups of light sources is done periodically, preferably every two images captured by the camera at a sampling frequency much higher than that of eye movements; this allows it to be done with sufficient frequency so that the rotation of the eye between both images is negligible, which allows successful results to be obtained.

In order to improve the estimation of the position of the flashes relative to each other and with respect to the absolute geometric reference, at least one of the following techniques can be used, alone or in combination, to produce a flash with a reduced position uncertainty, in order to determine an absolute geometric reference on the visible surface of the eye. The use of different techniques for determining this absolute geometric reference on the visible surface of the eye allows that, in the event that one of these techniques stops working, the system could use another technique, allowing the system to continue determining the absolute geometric reference on the visible surface of the eye.

In a first possible technique, at least one light source is configured to emit a light source of different geometric size than the rest of the plurality of light sources, the controller being configured to determine the absolute geometric reference on the visible surface of the eye identifying the flash in the image of the eye in relation to the corresponding light source configured to emit a light focus of different geometric size than the rest of the plurality of light sources.

For example, one way to configure the light source to emit a light focus of a different geometric size, in this case much smaller, than the rest of the plurality of light sources, would be to collimate the light beam emitted by said light source using a collimation mask that converts the divergent light beam coming from the light source into a parallel beam so that its geometric size decreases.

In a second possible technique at least one light source is configured to emit a light focus of different power than the rest of the plurality of light sources, the controller being configured to determine the absolute geometric reference on the visible surface of the eye identifying the flash in the image of the eye in relation to the light source configured to emit a light focus of different power than the rest of the plurality of light sources.

In a third possible technique: at least two light sources are closer to each other than the distance between the rest of the plurality of light sources, the controller being configured to determine the absolute geometric reference on the visible surface of the eye by identifying the flashes in the image of the eye in relation to light sources that are closer to each other than the distance between the rest of the plurality of light sources.

In a fourth possible technique, the system comprises a narrow beam light emitter, the controller being configured to determine the absolute geometric reference on the visible surface of the eye by identifying a reflection on the visible surface of the eye in relation to the narrow beam light emitter.

Preferably, said narrow beam light emitter would be centred with the optical axis of the camera, therefore improving the determination of the absolute geometric reference, with which the geometric relationship between the camera, the plurality of light sources and/or the narrow beam light emitter and the flares analysed in each image of the eye captured by the camera, thereby improving the generation of the 3D shape of the eye.

The main objective of the narrow beam light emitter is to produce a reflection smaller than the reflections of the plurality of light sources on the visible surface of the eye, either on the cornea (if the user is looking forward) or on the sclera (if the user looks to the side).

To this end, in one version of the present invention, the narrow beam light emitter emits an infrared laser.

In another version of the present invention, the narrow beam light emitter emits a beam of collimated diffuse infrared light.

This reflection can also be achieved with both technologies, since both emit a narrow beam of light that produces a point reflection much smaller than the reflections of the plurality of light sources.

This more punctual reflection is used by the present system both to determine the absolute geometric reference on the visible surface of the eye, and to improve the resolution of its position and use it to improve the position estimation of the other reflections of the plurality of light sources.

Not only that, the same narrow beam of light that produces the point reflection on the visible surface of the eye, penetrates the eye to its retina and produces a point reflection on the retina.

In the retina, the infrared laser or the collimated diffuse infrared light beam is reflected in diffuse mode: the collimated diffuse light beam because it is diffuse light from the start; and the infrared laser because the light loses coherence when reflected from the retina. The reflection on the retina, now diffuse, produces a spherical wave front that recoils until it emerges through the pupil.

The system will incorporate means to capture this spherical wave front and determine, based on the same, the instantaneous refraction of the eye (sphere, cylinder and axis).

Due to this, in this configuration, the narrow beam light emitter is also used as an auto refractometer, so that the reflection of the point that the laser produces on the retina allows the user's current accommodative state to be determined. This information, together with the 3D shape of the eye and the results of visual perceptual tests, also allows interpreting the effects of possible corneal alterations of the user, based on the results of the user's perception, their accommodative behaviour and their oculomotor skills.

It should be noted that when the narrow beam light emitter emits a laser, the system has to integrate this signal over time to improve the signal-to-noise ratio (due to the speckle phenomenon), while when the narrow beam light emitter emits a beam of diffuse collimated light, the system instantly calculates the measurement. Consequently, the latter can take instantaneous refraction measurements at a higher temporal frequency.

Additionally, the infrared characteristic of both narrow beam light emitter options is not visible to the user's eye, so the user does not suffer discomfort from the use of this narrow beam light emitter.

For this reason, preferably the light sources are infrared light-emitting diodes (LEDs), since with this configuration the user cannot see said light sources and, therefore, does not suffer discomfort, being able to continue performing their visual task with complete normalcy.

Preferably, the camera comprises optics associated with a variable focus system with focus control, which allows the camera to focus correctly on the visible surface of the eye, so that the images obtained by the camera are always in an optimal state.

Alternatively, the camera comprises optics associated with a fixed focus system with a field depth of at least 5 mm, both for depth variations in the relative position of the eye with respect to the absolute reference system produced by the user's movements and for the image of the eye captured by the camera to be always in focus.

The present system uses a matrix of light sources that allow the construction of a three-dimensional image of the eye (i.e., with depth), which is used in the calculation of gaze direction tracking. For the camera to capture this three-dimensionality, the field depth must have a minimum dimension of 5 mm.

### Description of the Figures

Figure 1 shows a cross-sectional view of an example of a human eye, detailing its main parts.
Figure 2 shows a cross-sectional view of an example of a human eye, detailing the difference between pupillary axis and gaze direction.
Figure 3 shows a view of the distribution of the light sources in the preferred embodiment of the invention.

### Detailed description of the invention

For a better understanding of the present invention, we will first briefly explain the different parts of the human eye.

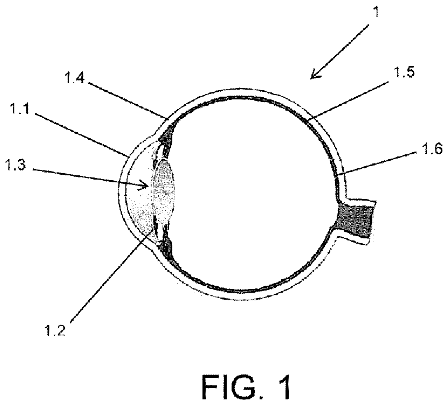

Figure 1 shows an example of a human eye (1), the function of which is to transform light energy into electrical signals that are sent to the brain through the optic nerve.

The visible outer surface of the eye (1) comprises the cornea (1.1), which is the transparent front portion of the eye (1) that covers the iris (1.2) and the pupil (1.3), and the sclera (1.4), called colloquially as the "white" of the eye (1), the sclera (1.4) is a white membrane, which makes up the outermost layer of the eye (1) and the function of which is to shape the eye (1) and protect the internal elements.

Likewise, on the inner surface of the eye (1) you can find the retina (1.5), when light hits the retina (1.5) a series of chemical and electrical phenomena are triggered, which translate into nervous impulses that are sent to the brain through the optic nerve. Likewise, the area of the retina (1.5) that is especially capable of accurate vision (greater visual acuity) and colour is called the fovea (1.6).

In a preferred embodiment of the present invention, the system comprises a plurality of light sources (2.1), more specifically infrared light-emitting diodes (LEDs), which emit infrared light towards at least one eye (1) of the user.

Since infrared light oscillates at a different frequency than the so-called "visible light", the human eye (1) is not able to see or perceive the same, so the user of the system does not suffer discomfort from the use of the plurality of light sources (2.1).

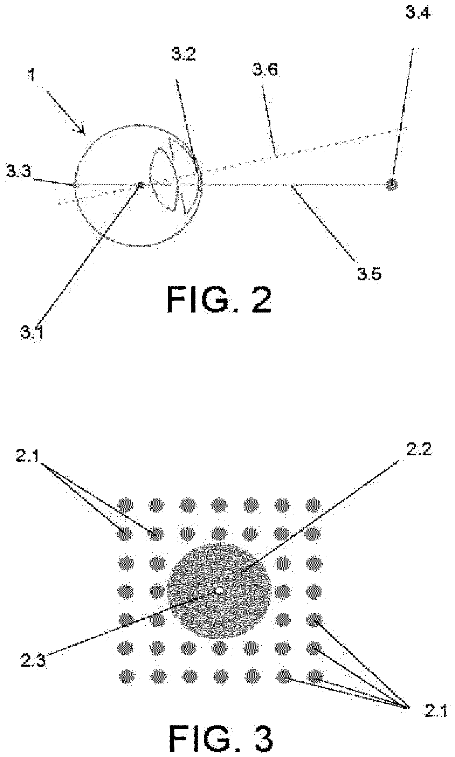

The plurality of light sources (2.1), preferably between 8 and 20, are arranged in the form of a matrix, the incidence of the plurality of light sources (2.1) covering, at least partly, or entirely, the visible surface of the eye (1), preferably around the camera (2.2) and fixed on the same frame, as can be seen in Figure 3, this allows a wide distribution of flashes on the visible surface of the eye (1) and avoids the problems caused by blinking or drooping eyelid in the state of the art. This distribution in the form of a matrix is not limiting and the plurality of light sources (2.1) can present any other distribution as long as they cover at least partly, or entirely, the visible surface of the eye (1).

This infrared light emitted towards the eye (1) of the user generates flashes on the visible surface of the eye (1), i.e., in the cornea (1.1) and/or sclera (1.4) of the eye (1), these flashes of infrared light are subsequently detected by a sensor capable of detecting this infrared light.

More specifically, in this exemplary preferred embodiment, the infrared sensor is a camera (2.2), capable of capturing images of the eye (1) and the corresponding flashes generated by the plurality of light sources (2.1).

The images obtained from the eye (1) captured by the camera (2.2) are analysed by a controller, which comprises at least one processor, so that this controller detects the different regions of the image of the eye (1) eye (for example, pupil, iris, sclera, eyelid, eyelashes or corners) and the plurality of flashes in the image of the eye (1).

In order to determine the geometric relationship between the camera (2.2), the plurality of light sources (2.1) and the flashes detected in the analysis of each image captured by the camera (2.2), the present system establishes an absolute geometric reference in the system made up of the camera (2.2), the plurality of light sources (2.1) (both in fixed relative positions) and an absolute geometric reference on the visible surface of the eye (1).

It is expected that to establish this absolute geometric reference on the visible surface of the eye (1) the system uses at least one of the following techniques, either alone or in combination:
- at least one light source (2.1) is configured to emit a light focus of different geometric size and/or power than the rest of the plurality of light sources (2.1), so that the flash generated on the visible surface of the eye (1) by said light focus is easily determined by the controller with reduced geometric uncertainty in the analysis of each image captured by the camera (2.2); and/or
- at least two light sources (2.1) are closer to each other than the distance between the rest of the plurality of light sources (2.1), and therefore the flashes generated on the visible surface of the eye (1) by said light sources (2.1) are easily determined by the controller in the analysis of each image captured by the camera (2.2); and/or
- the present system additionally comprises a narrow beam light emitter (2.3) that emits a narrow beam light beam (either an infrared laser beam or a collimated diffuse infrared light beam) towards the eye (1) of the user, of so that the flash generated on the visible surface of the eye (1) by said narrow beam light emitter (2.3) is easily determined by the controller with reduced geometric uncertainty in the analysis of each image captured by the camera (2.2).

In other words, the controller determines the absolute geometric reference on the visible surface of the eye (1) based on at least one flash easily determined by the controller in the analysis of each image captured by the camera (2.2).

The use of different techniques for determining the absolute geometric reference on the visible surface of the eye (1) allows that, in the event that one of these methods stops working, the use of the rest of the techniques would allow the system to continue determining the absolute geometry reference on the visible surface of the eye (1).

The controller, by determining the absolute geometric reference and knowing the correspondence between the plurality of light sources (2.1), the narrow beam light emitter (2.3) and the camera (2.2), determines in the analysis of each image captured by the camera (2.2) the geometric relationship or distance between the flash generated on the surface of the eye (1) and the light source (2.1) or narrow beam light emitter (2.3) that generated said flash.

Based on the correspondence between the absolute geometric reference, the flashes generated on the visible surface of the eye (1), the camera (2.2) and the absolute geometric reference on the visible surface of the eye (1), the controller generates a 3D shape of the eye (1) of the user and based on said 3D shape, the controller estimates the ocular centre of rotation (3.1) of the eye (1) by comparing two or more images of the eye (1) after a rotation.

As can be seen in figure 2, the ocular centre of rotation (3.1) indicates at which point the eye rotates (1) during the visual process, so its correct calculation is of the greatest importance for calculating the gaze direction (3.5).

Once the 3D shape of the eye (1) and the ocular centre of rotation (3.1) are determined, the controller can easily determine and take into account in the calculations the changes in the relative position of the eye (1) with respect to the camera (2.2) and, also, determine if the change in relative position is the cause of any adverse effects on the image quality of the camera (2.2). In this case, the controller may increase or decrease the power of the plurality of light sources (2.1) (for example, more power, if required, when the eye (1) moves away from the camera (2.2) and vice versa) and/or activate the associated optics of the camera (2.2), if it has variable focus.

In order to maintain image quality, the camera optics (2.2) are expected to be defined with a field depth of at least 5 mm and may include, in some configurations, a variable focus system with focus control.

This associated optics allows the controller to ensure that the images of the eye (1) captured by the camera are continually sufficiently focussed.

This determination of the ocular centre of rotation (3.1) is critical for the calculation of the gaze direction (3.5), which is defined as the geometric line that joins the active PRL (3.3) (normally present in the fovea (1.6) of the eye (1), although in certain ophthalmic pathologies it is located outside the fovea (1.6)) with the ocular centre of rotation (3.1).

As can be seen in Figure 2, this gaze direction (3.5) has no relationship with the position of the centre (3.2) of the pupil (1.3).

It should be noted that the active PRL (3.3) is a given group of retinal neurons (1.5) (whether or not they are in the fovea (1.6)) and that, therefore, its geometric position moves in solidarity with the eye (1).

It should also be noted that a user can use more than one PRL (3.3), each in its position integral with the eye (1), but there can only be one PRL (3.3) active at each instant. This implies that the gaze direction (3.5), i.e., the line that joins the active PRL (3.3) with the ocular centre of rotation (3.1), changes when the user changes the PRL (3.3).

Figure 2 shows an example of the difference between the pupillary axis (3.6), which is the geometric line that joins the centre of the pupil (3.2) with the ocular centre of rotation (3.1), and the gaze direction (3.5).

The improvement obtained with the proposed system reduces the measurement error of the gaze direction (3.5) compared to the state of the art by more than 0.5 degrees. It should be noted that, in clinical applications, it is interesting to measure displacements of the gaze direction (3.5) of 3 seconds of arc, which are the dimension of the drift paths during a fixation.

As previously mentioned, fixation is the visual process that allows a subject to maintain the projection of an external visual stimulus on his fovea (1.6) and, through a continuum of small involuntary ocular (combination of drift movements and microsaccades) movements (rotations), extract visual information related to its content in spatial frequencies.

Since a 1 degree angle contains 3,600 arcseconds, an error of 0.5° implies an error of 1,800 arcseconds, i.e., an error 600 times greater than the magnitude we are interested in measuring.

It should also be noted that, in clinical applications, it is interesting to measure accelerations of the gaze direction (3.5) of up to 800 degrees per second squared (even, possibly higher) that induce errors, in the state of the art, due to the inertial movements of the pupillary sphincter in relation to the eye (1) of up to an additional 30 arc minutes (1,800 arc seconds).

For all these reasons, the system described, combined with cameras (2.2) available in the state of the art with a temporal resolution of 3 ms (milliseconds), i.e., with more than 330 Hz sampling frequency, and with a spatial resolution of 1 minute of arc, represents a substantial improvement in each of these errors.

In order to determine the position of the active PRL (3.3) of the eye (1) at a given time, the controller is configured to detect characteristic movements of the eye (1) during a fixation on a series of images of the eye (1) captured by the camera (2.2) and calculate the angular position of a point outside (3.4) the eye (1) that determines the gaze direction (3.5). These fixation movements are involuntary and occur exclusively when there is a stimulus on which the user can fix their gaze. If, for example, this stimulus is unique in the user's virtual visual space and the user's eye (1) shows fixation movements, it can be concluded that the user's eye (1) is fixating on that stimulus.

To carry out this fixation, the eye (1) must rotate with a characteristic movement called drift, interrupted by other movements called microsaccades that reposition the stimulus image on the active PRL (3.3).

The drift consists of small position jumps of a few arc minutes (3 to 30) that develop consecutively with a "random-walk" type statistic with short periods of persistent direction, followed by short periods of anti-persistent direction.

Microsaccades are position jumps an order of magnitude larger and ballistic in nature (i.e., with a constant ratio of maximum velocity and amplitude).

In total, a typical fixation lasts between 150 ms and 250 ms.

If the system can measure fixation movements (drift and microsaccades) and if the stimulus is small (at the limit of the user's spatial visibility), the system will be able to determine the direction of the point outside (3.4) the eye (1) (the distance to the eye (1) of which is not relevant for these calculations) during a fixation, defined as the line of gaze direction (3.5), connecting the ocular centre of rotation (3.1) with the position of a central measure of the statistical distribution of the ocular rotations during that fixation (for example, it is determined that the point outside (3.4) the eye (1) is located in the barycentre of the rotations).

With this, the controller is able to estimate an active PRL (3.3) of the eye (1) in the 3D shape generated based on the projection to the rear portion of the eye (1), where the fovea (1.6) and/or retina are located. (1.5), of the axis of the gaze direction (3.5) that connects the ocular centre of rotation (3.1) and the point outside (3.4) the eye (1).

The same calculation process will be able to detect an active PRL change (3.3) and update the calculation of the gaze direction (3.5).

The calculation process of the controller will associate the 3D coordinates of this active PRL (3.3) to the generated 3D shape, so that, from that moment on, the gaze direction (3.5), at each instant, will be determined by the geometric line that connects that active PRL (3.3) with the ocular centre of rotation (3.1).

For all these reasons, this system does not require prior calibration, although, to accommodate anomalous behaviour in cases of certain visual dysfunctions, a statistical treatment of post-processing data is planned.

Likewise, the plurality of light sources (2.1) comprises at least two groups of light sources (2.1) configured to emit light alternately. For this purpose, the plurality of light sources (2.1) have variable light intensity (with at least 2 levels) through individual electronic control of each light source (2.1) or by groups of light sources (2.1).

This alternation between groups of light sources (2.1) is such that at any given time all light sources (2.1) of one group are active (turned on), while all light sources (2.1) of the other are inactive (turned off).

This alternation is controlled so that for every two consecutive images of the camera (2.2), one image will have been obtained with the first group of light sources (2.1) activated and the second group of light sources (2.1) deactivated and the second image with the second group of light sources (2.1) activated and the first group of light sources (2.1) deactivated.

This makes it possible to improve the image of the pupil edge (1.3) in the images of the eye (1) captured by the camera (2.2), since, if, in an image, an area of the pupil edge (1.3) is difficult to distinguish as it is hidden by the flash of a light source (2.1) of the first active group, good visibility of that area of the edge of the pupil (1.3) is assured when the second group of light sources (2.1) is activated immediately afterwards.

Figure 3 shows a view of the distribution of the light sources (2.1), the camera (2.2) and the narrow beam light emitter (2.3) in the preferred exemplary embodiment of the invention.

More specifically, it can be observed how the narrow beam light emitter (2.3) emits a narrow beam of light, the reflection on the eye (1) of which has less position uncertainty than the flashes of the plurality of light sources (2.1), in correspondence with the axis of the camera (2.2), so as to allow a better calculation of the absolute geometric reference on the surface of the eye (1). Additionally, in a preferred configuration, the narrow beam light emitter (2.3) is used as the light source of an autorefractometer.

Likewise, Figure 3 shows how the plurality of light sources (2.1) are arranged in the form of a matrix around the camera (2.2) and at different distances from each other vertically and horizontally, this allows a wide and relatively constant distribution of flashes on the visible surface of the eye (1) and reduces problems caused by blinking or drooping eyelids, since flashes continue to be seen even under these conditions.

## Claims

1. A system for tracking a user's gaze direction (3.5), which comprises:
- a plurality of light sources (2.1) that emit infrared light onto at least one eye (1) of the user,
- a camera (2.2) configured to capture infrared images of the eye (1);
- a controller comprising at least one processor, wherein the controller is configured to:
∘ detect a plurality of flashes in an image of the eye (1), captured by the camera (2.2), each flash being a reflection of one of the light sources (2.1) on the visible surface of the eye (1); and
∘ to match each detected flash to the light source (2.1) that generated the flash on the visible surface of the eye (1);
**characterised in that**:
- the plurality of light sources (2.1) is arranged in the form of a matrix covering, at least partly, the visible surface of the eye (1); and
- the controller is additionally configured to:
∘ analyse the image of the eye (1) and to determine to which region of the eye (1) each pixel of the image belongs;
∘ generate a 3D shape of the eye (1) from the plurality of flashes relative to an absolute geometric reference determined by the position of the camera (2.2) and the plurality of light sources (2.1); and
∘ define an ocular centre of rotation (3.1) of the eye (1) on the basis of the 3D shape of the eye (1), with which the gaze direction (3.5) is determined.

2. The tracking system, according to any one of the preceding claims, **characterised in that** the controller is additionally configured to:
- detect characteristic eye movements (1) in a series of eye images (1) captured by the camera (2.2) during a gaze fixation;
- estimate a point outside (3.4) the eye (1) of the gaze direction line (3.5) on the basis of the characteristic movements of the eye (1) in said fixation;
- define an active PRL (3.3) of the eye (1) in the 3D shape of the eye (1) as the point of intersection with the rear portion of the 3D shape of the eye (1) of the gaze direction (3.5) line defined by the ocular centre of rotation (3.1) and the point outside (3.4) the eye (1).

3. The tracking system, according to the preceding claim, **characterised in that** the plurality of light sources (2.1) comprises at least two groups of light sources (2.1) configured to emit light alternately.

4. The tracking system, according to any one of the preceding claims, **characterised in that** at least one light source (2.1) is configured to emit a light focus of different geometric size than the rest of the plurality of light sources (2.1), the controller being configured to determine the absolute geometric reference on the visible surface of the eye (1) identifying the flash in the image of the eye (1) in relation to the corresponding light source (2.1) configured to emit a light focus of different geometric size than the rest of the plurality of light sources (2.1).

5. The tracking system, according to any one of the preceding claims, **characterised in that** at least one light source (2.1) is configured to emit a light focus of different power than the rest of the plurality of light sources (2.1), the controller being configured to determine the absolute geometric reference on the visible surface of the eye (1) identifying the flash in the image of the eye (1) in relation to the light source (2.1) configured to emit a light focus of different power than the rest of the plurality of light sources (2.1).

6. The tracking system, according to any one of the preceding claims, **characterised in that** at least two light sources (2.1) are closer to each other than the distance between the rest of the plurality of light sources (2.1), the controller being configured to determine the absolute geometric reference on the visible surface of the eye (1) identifying flashes in the image of the eye (1) in relation to the light sources (2.1) that are closer to each other than the distance between the rest of the plurality of light sources (2.1).

7. The tracking system, according to any one of the preceding claims, **characterised in that** it comprises a narrow beam light emitter (2.3), the controller being configured to determine the absolute geometric reference on the visible surface of the eye (1) identifying a reflection on the visible surface of the eye (1) in relation to the narrow beam light emitter (2.3).

8. The tracking system, according to the preceding claim, **characterised in that** the narrow beam light emitter (2.3) emits an infrared laser.

9. The tracking system, according to claim 7, **characterised in that** the narrow beam light emitter (2.3) emits a beam of diffuse collimated infrared light.

10. The tracking system, according to any one of the preceding claims, **characterised in that** the light sources (2.1) are infrared light-emitting diodes (LEDs).

11. The tracking system, according to any one of the preceding claims, **characterised in that** the camera (2.2) comprises optics associated with a variable focus system with focus control.

12. The tracking system, according to any one of claims 1 to 10, **characterised in that** the camera (2.2) comprises optics associated with a fixed focus system with a field depth of at least 5 mm.
